# EUROPEAN PATENT SPECIFICATION

(11) **EP 2 999 391 B1**
(45) Date of publication and mention of the grant of the patent: **17.10.2018**
(21) Application number: 14801521.7
(22) Date of filing: 21.05.2014
(51) Int. Cl.: A61B 1/00

(54) **AN ENDOSCOPE ACCESSORY**
ZUBEHÖR FÜR EIN ENDOSKOP
ACCESSOIRE D'ENDOSCOPE

(30) Priority: 22.05.2013 US 201313900524
(43) Date of publication of application: 30.03.2016
(73) Proprietor: Farhadi, Ashkan, Irvine, California 92603 (US)
(72) Inventor: Farhadi, Ashkan, Irvine, California 92603 (US)
(74) Representative: Maikowski & Ninnemann Patentanwälte Partnerschaft mbB
(86) International application number: PCT/US2014/038929
(87) International publication number: WO 2014/190026

(56) References cited:
- JP-A- 2013 075 101
- JP-A- 2013 075 101
- US-A- 5 217 001
- US-A- 5 916 145
- US-A1- 2005 101 837
- US-A1- 2010 121 144
- US-A1- 2010 121 144
- US-A1- 2011 105 840
- US-A1- 2011 251 458
- US-A1- 2013 281 781

## Description

### FIELD OF INVENTION:

This invention relates generally to an accessory for endoscopic examination of body organs, particularly the gastrointestinal tract. More so, the invention relates to the creation of an examination compartment around the tip of an endoscope introduced into a visceral organ.

### BACKGROUND OF INVENTION:

An endoscope is a well-known optical system for evaluation of internal organs disclosed and claimed in U.S. Pat. No. 3,449,037 to C. J. Koester. Currently used fiber optic endoscopes are comprised of lenses mounted in a flexible tube to relay an image from inside a body cavity for viewing by a physician for diagnosis or manipulation inside cavitary spaces. An echoendoscope (EUS) is a device that combines endoscopy and ultrasound to image the gastrointestinal wall and surrounding structures. The first prototype for human use was developed in 1980, and several generations of echoendoscopes have been developed since then. In the 1990s, the capability of obtaining tissue samples by this method resulted in further applications of this test to sample internal structures and organs. The ultrasound transducer is positioned at the tip of endoscope and the key components of the transducer are the piezoelectric crystals that vibrate to produce ultrasonic waves. The ultrasonic waves travel through gastrointestinal wall and beyond the visceral wall into the surrounding organs. The reflection of these ultrasound waves will be detected by the same crystals at the transducer and reconstruction of these reflections will result in creating a real time image of the gastrointestinal wall and its surrounding structures. The ultrasonic wave reflects from the surface of structures with different density and can pass very well through fluid containing and solid structures. Air, however, creates a barrier to ultrasonic wave passage and hampers the obtaining of ultrasonic images. Many attempts have been done to minimize the amount of interfering air between the transducer and the examining structure. These efforts could be seen in early patents by Yokoi in 1988 (Ultrasonic endoscope, U.S. Pat. No. 4,779,624), Wollschlager in 1992 (Ultrasound endoscope device, U.S. Pat. No. 5,105,819), Sakamoto in 1994 (Ultrasound transmission medium feed device for endoscopically inserting ultrasound probe, U.S. Pat. No. 6,004,273) and recently in the patent application by Nierich in 2007 (Transmission device for ultrasonic imaging system, publication No 2007/0038109). In all of these, there is balloon at the end of the endoscope that encloses the transducer and is filled with water to permit acoustic coupling between the transducer and the luminal wall or other gastrointestinal structures. This is particularly helpful in the part of the gastrointestinal tract where the diameter of the lumen is small and the inflated balloon makes good circumferential contact with the intestinal wall and thus creates a good acoustic coupling. In most parts of the gastrointestinal tract, however, the large diameter of the lumen and/or the angle of the transducer in relation to the intestinal wall results in an inadequate contact between the transducer balloon and the intestinal wall. Therefore, the operators usually use water infusion to fill the region of the gastrointestinal tract with water and create acoustic coupling between the transducer and the examined structures. Unfortunately, the gastrointestinal tract is not a closed region and the infused water soon moves to other regions of the gastrointestinal tract. This can often result in poor image quality despite repeated infusion of water around the transducer. In addition, infusion of significant amounts of water during the examination could result in untoward problems such as aspiration of the water into the patient's airway or overdistention of the gastrointestinal tract. To overcome this problem, I have devised a device that creates a closed space around the ultrasound transducer using balloons. The use of balloons in the gastrointestinal system was suggested for the first time by Wilcox in 1987 (Double balloon nasobiliary occlusion catheter for treating gallstones and method of using the same U.S. Pat. No. 4,696,668) who used a double balloon catheter to make a closed space inside the bile duct to direct the chemicals used for lysing of gall bladder stone into the gall bladder and limit the exposure of the rest of the biliary system with this toxic agent. Later, a two-balloon approach was used on various endoscopic devices for assisting the movement of the endoscope deep down into the small intestine. The initial device was proposed by Fujikura in 2005 (Insertion assisting tool for endoscope, publication No 2005/0124856), Takano in 2005 (Endoscope apparatus, publication No 2005/0165273), Machida in 2005 (Endoscope apparatus, publication No 2005/0215855), and Yoshida in 2007 (Double-balloon endoscope system, publication No 2007/0049797). In all of these patents, an overtube with a balloon is used to secure the position of the endoscope inside the gastrointestinal tract and the second balloon on the inserting tip of the endoscope is used to anchor and move the endoscope forward using alternating inflating and deflating of these two balloons.

Another device was described in U.S. Publication No. 2005/0107664 to Kalloo et al. which proposes an elongated dilating balloon that serves to provide a uniform dilation of the stomach wall and two inflatable balloons affixed to the distal end of the overtube. Inflation of these two balloons can create a seal around the overtube while the overtube passes through a body cavity. The seal created by these two balloons can act like a barrier between two body cavities in this case between stomach and intra-abdominal cavity (peritoneum). In contrast to Kalloo's design, where two balloons are located at the distal end of the overtube, in the claimed device an inflatable positioning ring is affixed to the overtube at the distal end and an occlusion balloon is affixed a catheter at the distal end. The catheter passes through a passageway defined by the overtube. In addition, our overtube has a longitudinal seam that allows the overtube to be placed over the shaft of the endoscope. In Kalloo's device, the balloon's function is to create a seal between two cavities (gastrointestinal lumen and peritoneal cavity). The present device, however, creates a sealed region in a single cavity (the gastrointestinal lumen). In Kalloo's device both balloons are in a fixed position, but in the claimed design, the positioning of the sealing devices are independent of one another. In addition, an elongated dilating balloon can be used to help pass the overtube through a cut that is made in the gastrointestinal lumen. This dilating balloon passes through a passageway that is located within the endoscope and differs from the claimed occlusion balloon at the end of a catheter that passes through a passageway defined by the overtube. This gives this occlusion balloon freedom to move independently of the endoscope. In fact, there is no need to have an endoscope to deploy the occlusion balloon. The dilating balloon in Kalloo's device can only be introduced if the endoscope has already placed inside the overtube. In addition, in Kalloo's design, the dilation balloon is just an accessory that is needed to temporarily dilate the opening of the incision in the stomach to help the passage of the overtube through the opening.

Another device was proposed by Chang US Patent 6,712,755B2 in 2004. This device comprises of a locking mechanism for an endoscopic overtube comprising an interdigitaing configuration with a plurality of interlocking members that join together to close the overtube. Obviously, this device can act as an overtube that can envelop around the shaft of the endoscope. However, it does not have any positioning balloon for securing the position within the gastrointestinal lumen, it does not use a magnet or an adhesive for closure and it has special interlocking members that need to be aligned and locked using a fastener.

Another device was proposed application 20090227835 by Terliuc. This device is a flexible small overtube that is assembled on the distal shaft of an endoscope and then travels with the endoscope inside the body cavity. This overtube is fixed over the shaft of the endoscope and cannot be placed or moved independent of the endoscope in opposed to my invention, which allows the endoscope to move freely and independently inside the overtube. In fact, the endoscope can even be replaced with another endoscope while the overtube can be kept in its position. In addition, the overtube proposed by Terliuc cannot be moved independently of the endoscope inside the body cavity since the proximal end of the overtube is also inside of the body cavity, while my claimed invention has a proximal end that is situated outside of the body cavity and can be moved independently of the endoscope. Not to mention that my overtube has a longitudinal seam that allows the overtube to be placed over the shaft of the endoscope.

Another device is shown in Wenner et al. U.S. Patent 6,440,061. Wenner et al. proposed an overtube with a catheter situated within a catheter tube and a free, independently positionable distal end portion that terminates in an inflatable occlusion balloon that exits from the side of the flexible overtube, but not the end of the flexible overtube. Thus, it cannot create a liquid tight space at the end of the overtube. This is because the occlusion balloon in Wenner's patent is situated at the same side as the positioning balloon in relation to the end of the endoscope. In contrast, the present claims define an occlusion balloon and a positioning ring independently positioned on the proximal and the distal positions in relation to the end of the endoscope, thereby creating a liquid tight space around the end of the endoscope. In fact, if the occlusion balloon of Wenner's invention was to exit from the end of the flexible overtube and placed distal to the end of the endoscope the purpose of their invention, access of endoscope to the site of interest would be prevented since the endoscope view and access would have been blocked by the occlusion balloon. In addition, passage of a flexible tube into body cavity needs a rigid introducer sheath, while in my invention the overtube is being inserted into the body cavity over the endoscope and uses endoscope as the guide. The overtube also has a curved lower distal end and an angled tip, which is designed to work in an acute angled spot such as bile duct. This prevents this device to work similar to my device, which has a straight distal end in the gastrointestinal (GI) tract. Also, there is no structure in the overtube that can create a hydraulic seal between the endoscope and overtube. Thus, fluid can leak around the endoscope since the device cannot create nor maintain a hydraulic sealed area distal to the tip of the overtube between the positioning balloon and the occlusion balloon similar to the current invention. Wenner's device uses two occlusion balloons called first and second occlusion balloon that both can pass through overtube sheath ports to create a hydraulically sealed area between these two balloons, but the hydraulically isolated region is not between an occlusion balloon and a positioning ring at the distal of the overtube. Not to mention that the device is not designed to achieve a hydraulically sealed region due to the lack of any structure in the overtube to seal the endoscope within the overtube. Finally, the present overtube has a longitudinal seam that allows the overtube to be placed over the shaft of the endoscope.

Yet another device was proposed by Chu et al. U.S. Patent 5,916,145. In this device a flexible mesh overtube is inserted with the use of endoscope shaft as a guide and a distal housing that includes a suction chamber and an endoscope chamber on its distal end. Between the endoscope chamber and the suction chamber, there is a clear window, which allows the endoscopic view of the area distal to the overtube through this window. The endoscope tip does not exit the distal end of overtube. This allows the endoscope to stay away from contaminants per inventors' point of view. The only access to the body cavity is through a passageway, which allows use of tools such as forceps or catheters distal to the tip of the overtube. A fundamental difference between this device and the current invention is that the endoscope exits the distal end of the overtube into body cavity in the current invention. In fact, for devices such as echoendoscope, it is essential for the distal tip of endoscope to touch the lining of the body cavity. Moreso, the Chu overtube is not a through passageway for endoscope, and the entire mesh-like mechanism works when the endoscope at the end pulls the distal end inside the body cavity by pushing force over its shaft. In addition, Chu et al. device does not have a catheter that terminates in an inflatable occlusion balloon as an integral part of the described overtube. There is mention of a possibility of using a dilation catheter that can be positioned in the distal end. The Chu et al. device fails to teach a structure that creates a seal inside the overtube and around the endoscope since the distal end of the endoscope at most abuts clear window or if there is no clear window, the tip of the endoscope cannot go beyond the overtube. In addition, the present overtube has a longitudinal seam that allows the overtube to be placed over the shaft of the endoscope.

A similar endoscope accessory is disclosed in US 2013/0281781 and in US 2010/0121144.

In the current invention, on the other hand, the structure of the balloons and their functions are different.

### SUMMARY OF THE INVENTION

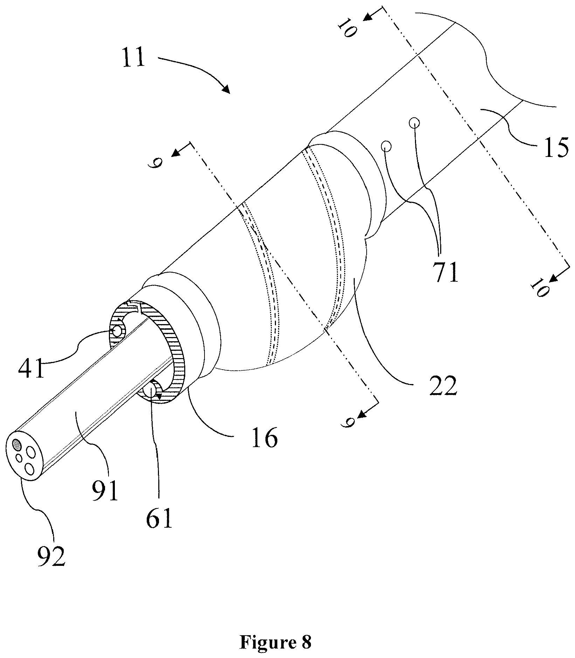

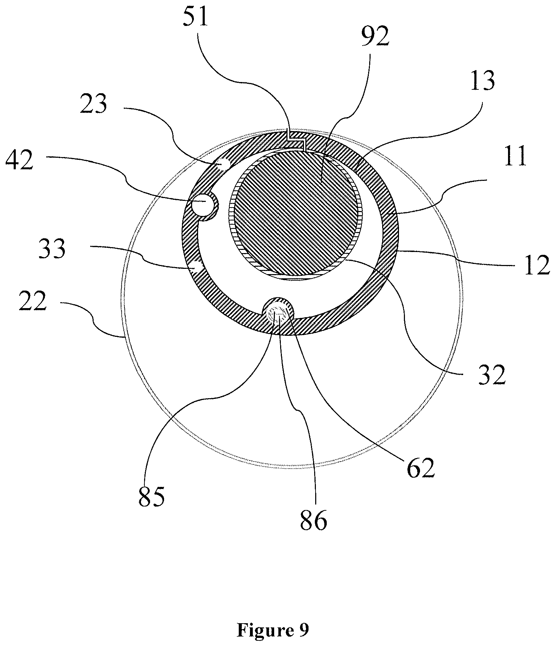

The endoscope accessory embodying this invention enhances capabilities of an endoscope in maintaining luminal view and is defined by claim 1. The inventive feature can best been seen in figures 8 and 9. The endoscope accessory is comprised of a flexible elongated sheet, sized to envelop a flexible endoscope or echoendoscope shaft and envelop an endoscope or echoendoscope shaft without need to remove endoscope from the body when its opposed longitudinal edge portions coacting to form a flexible overtube receiving therewithin the endoscope or echoendoscope shaft. The longitudinal edges of the endoscope accessory overlap forming a seam and are supplied with an adhesive along its entire length for coacting with the opposed edge portions to form a liquid tight seam along the entire length of the overtube. The adhesive is covered by a release sheet that can be removed before adhering the two longitudinal end portions. Alternative to use adhesive at the longitudinal edges of the endoscope accessory for creating the longitudinal seam, the overtube seam can be closed along its entire length using a plurality of spaced magnets on the longitudinal edge portions of the endoscope accessory sheet that are attracting the other magnets interspersed on the other longitudinal edge portion. In order to avoid activation of the magnets before placing the endoscope shaft within the overtube, at least one longitudinal edge portion is supplied with a longitudinal magnet cover. Preferably, both longitudinal edge portions are provided with a cover. The magnets can only coact with the opposed longitudinal edge portion magnets when the magnet covers are removed from the edge portion or portions.

The overtube is provided with a handle on the external surface at the proximal end of the overtube for grasping and manipulation of the overtube within the body cavity. The handle is also the hub where a plurality of external inflation tubes and ports can connect to the overtube and can be used for connection of the overtube to inflation device, suction device or passage of therapeutic tools and catheters through the overtube.

The overtube is further provided with an inflatable positioning ring on external surface of the flexible overtube for securing the position of the overtube within the body cavity. The inflated positioning ring is asymmetric or eccentric in regard to the overtube. This allows a better sealing created by inflatable positioning ring within the body cavity and also improves the maneuverability of the endoscope tip within the body cavity.

The overtube is further provided with a positioning ring inflation tube in communication with the inflatable positioning ring for inflating or deflating the inflatable positioning ring.

The overtube is further supplied with an inflatable sealing band on the internal surface of the overtube at either the distal, mid or proximal portion of the overtube for creation a seal around the endoscope shaft within the overtube. Alternatively, the inflatable sealing band can be replaced with an elastomeric sealing band on the internal surface of the flexible overtube. The inflated sealing band is eccentric to the overtube. This allows a better seal created by inflatable sealing band over endoscope.

The overtube is further provided with a sealing band inflation tube for inflating or deflating the inflatable sealing band.

The overtube is further provided with a catheter passageway which defines a passageway for passing catheter or other accessories such as biopsy forceps and other endoscopic accessories from the overtube proximal end portion to the distal overtube end portion.

The endoscope accessory is also provided with an occlusion catheter carried by the overtube through the catheter passageway, having a free, independently positionable distal end portion that terminates in an inflatable occlusion balloon.

The occlusion catheter is composed of an occlusion balloon port for inflating or deflating of the inflatable occlusion balloon and also a suction port that terminates in a suction tip downstream of the occlusion balloon that facilitates removal of secretions of body cavity distal to the occlusion balloon.

The overtube is further provided with a fluid conduit that defines a passageway for inflating or suctioning fluid or air within the examination compartment within the body cavity at the distal end of the overtube.

The overtube is further provided with a suction conduit connected to a suction port situated on the external surface of the overtube between the inflatable positioning ring and the proximal end portion of the overtube. The suction port can be used to suction the secretions that accumulate proximal to the inflatable positioning ring in the body cavity.

In use, the endoscope accessory of the claimed invention is a flexible, elongated sheet that envelops an endoscope or echoendoscope shaft while the endoscope shaft is still within the body cavity without the need to remove the overtube or pre-position the overtube over the endoscope shaft prior to the endoscope insertion within the body cavity. After enveloping or surrounding the endoscope shaft by the sheet, the opposing longitudinal edge portions are joined to form an overtube by creating a longitudinal seam along the entire length of the overtube. The opposed longitudinal edge portions of the sheet are supplied with adhesive and covered by a release sheet. To form the overtube, the release sheet is removed and the opposed longitudinal edge portions overlapped and bonded by adhesive to form a liquid tight seam. Alternatively, the longitudinal edge portions can be supplied with plurality of spaced magnets covered by a magnet cover comprised of a slit sleeve and the like. The magnets on the longitudinal edge portions are able to coact upon removal of the magnet cover. The magnet pieces on one edge portion are interspersed with the spaced magnets on the other longitudinal edge portion to form the seam. Alternatively, the seam can be created by other interlocking mechanisms such as tongue and groove, hook and loop, or zip-lock-type fastener and the like.

After closure of the overtube seam along the overtube's entire length, the endoscope shaft is enveloped within the overtube, the handle of the overtube is grasped and the overtube is pushed into the body cavity of a patient with the guide of the endoscope shaft to be placed at the desired location, just proximal to the tip of the endoscope.

Then, the inflatable positioning ring is inflated to secure the position of the overtube distal end portion within the body cavity.

The inflatable sealing band is then inflated to secure the position of the endoscope within the overtube and create a seal around the endoscope shaft. The endoscope can be replaced prior to inflating the inflatable sealing band, if desired, with another endoscope while the overtube stays in its position within the body cavity.

The occlusion balloon catheter is passed through the catheter passageway to be placed beyond the distal tip of the overtube. After the inflatable occlusion balloon exit the catheter passageway at the distal tip of the overtube, it is placed at the desired location, distal to the distal tip of the endoscope. Then, the inflatable occlusion balloon is inflated.

Inflation of the occlusion balloon creates an examination compartment defined by the inflated positioning ring, inflated sealing band and the inflated occlusion balloon at the tip for the endoscope at the distal end of the overtube. The examination compartment can be filled with air, or with water, depending on applications. The inflated balloons prevent escape of air or water from the examination compartment.

Throughout the procedure, the pressure within the examination compartment is maintained and monitored through the fluid conduit.

After termination of the examination, the positioning ring, sealing band and occlusion balloon are all deflated and the overtube is removed independent of the endoscope.

### BRIEF DESCRIPTION OF THE DRAWINGS:

Figure 1 is a schematic illustration of the the endoscope accessory within the upper gastrointestinal tract.
Figure 2 is a plan view of the present invention.
Figure 3 is a sectional view of the device shown in Figure 2 taken along plane 3-3.
Figure 4 is a longitudinal sectional view of the device shown in Figure 2 taken along plane 4-4.
Figure 5 is a schematic illustration of the the endoscope accessory before placement of the endoscope shaft.
Figure 6 is a schematic illustration of the the endoscope accessory after placement of the endoscope shaft and closure of the longitudinal seam.
Figure 7 is a schematic illustration of the the endoscope accessory after placement of the endoscope shaft, closure of the longitudinal seam and inflation of the inflatable positioning ring.
Figure 8 is another schematic illustration of the the endoscope accessory from different perspective after placement of the endoscope shaft, closure of the longitudinal seam and inflation of the inflatable positioning ring.
Figure 9 is a sectional view of the device shown in Figure 8 taken along plane 9-9.
Figure 10 is a sectional view of the device shown in Figure 8 taken along plane 10-10.
Figure 11 is a partial plan view of the present invention showing spaced magnets and magnet covers at the longitudinal edge portions as an alternative embodiment;
Figure 12 is a sectional view of the present invention showing spaced magnets and magnet covers at the longitudinal edge portions as an alternative embodiment device shown in Figure 11 taken along plane 12-12.
Figure 13 is a schematic illustration of the endoscope accessory showing spaced magnets and magnet covers at the longitudinal edge portions as an alternative embodiment before placement of the endoscope shaft.
Figure 14 is a schematic illustration of the the endoscope accessory showing spaced magnets and magnet covers at the longitudinal edge portions as an alternative embodiment after placement of the endoscope shaft and partial closure of the longitudinal seam.
Figure 15 is a partial sectional view of the present invention showing tongue and groove mechnism at the longitudinal edge portions as an alternative embodiment shown in Figure 2 taken along plane 3-3.
Figure 16 is a sectional view of the present invention showing an elastomeric bead as an alternative to inflatable sealing pocket shown in Figure 2 taken along plane 3-3.
Figure 17 is a longitudinal sectional view of the present invention showing the elastomeric bead as an alternative to inflatable sealing pocket shown in Figure 2 taken along plane 4-4.
Figure 18 is a sectional view of the present invention shown in Figure 8 taken along plane 9-9, showing the elastomeric bead forming an elastomeric sealing band.
Figure 19 is a schematic illustration of the occlusion balloon catheter.
Figure 20 is a schematic illustration of the endoscope accessory within the lower gastrointestinal tract.

### DETAILED DESCRIPTION OF THE INVENTION:

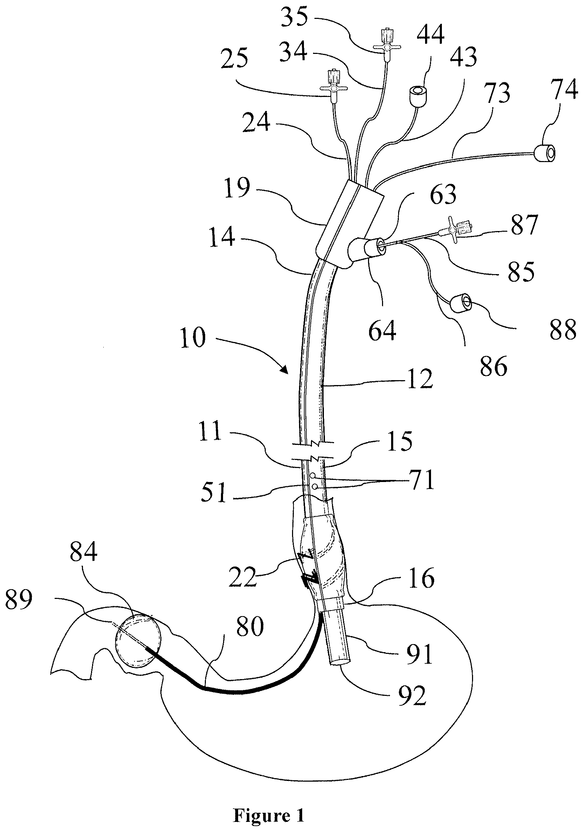

Endoscope Accessory 10 has the following components:
**A-Overtube:** As it is depicted in Figure 1, an overtube 11 defines a central through passageway for receiving therewithin an endoscope or echoendoscope shaft 91 and then is inserted inside a body cavity such as gastrointestinal tract.

The overtube 11 has an external 12 surface and an internal surface (not shown in figure 1), a proximal end portion 14, a midportion 15 and a distal end portion 16. The overtube 11 is also supplied with a handle 19 at its proximal end portion 14. Several catheters exit from the handle 19. These catheters are used for connection to inflation devices, suction devices or passage of therapeutic tools through the overtube 11.

The length of the overtube 11 is long enough so that when the distal overtube end portion 16 is secured inside the body cavity, the overtube proximal end portion 14 stays out of the body cavity and allows grasping of the handle 19 and manipulation of the overtube 11 for proper positioning of the overtube distal end portion 16 within the body cavity by the endoscopist. The diameter of the overtube 11 is wide enough to freely receive a regular endoscope or echoendoscope shaft 91 therewithin. Within the body cavity, the endoscope tip 92 extends beyond the overtube 11 distal end portion 16 (Figure 1) for detailed examination of the body cavity. The overtube 11 has a longitudinal seam 51 along its entire length that allows opening of the overtube 11 along its entire length for placing the endoscope shaft 91 within the overtube 11 without the need for removing the endoscope shaft 91 from the body cavity.

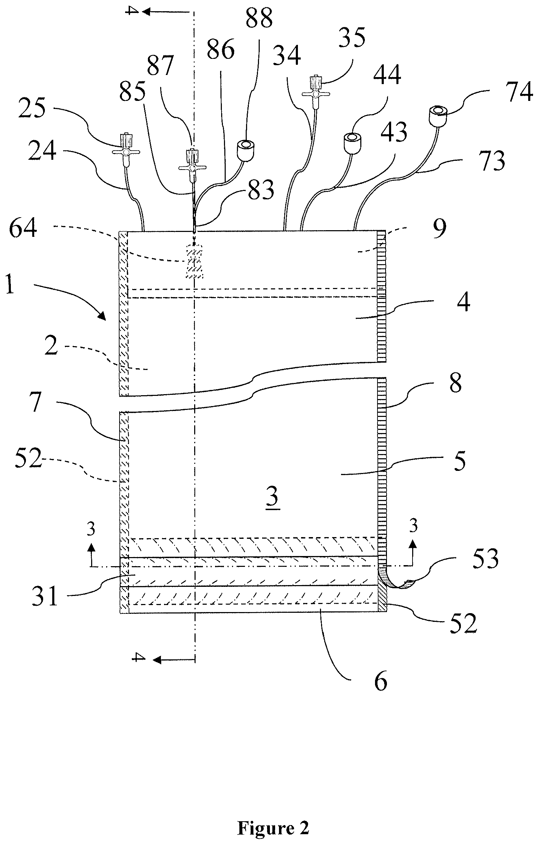

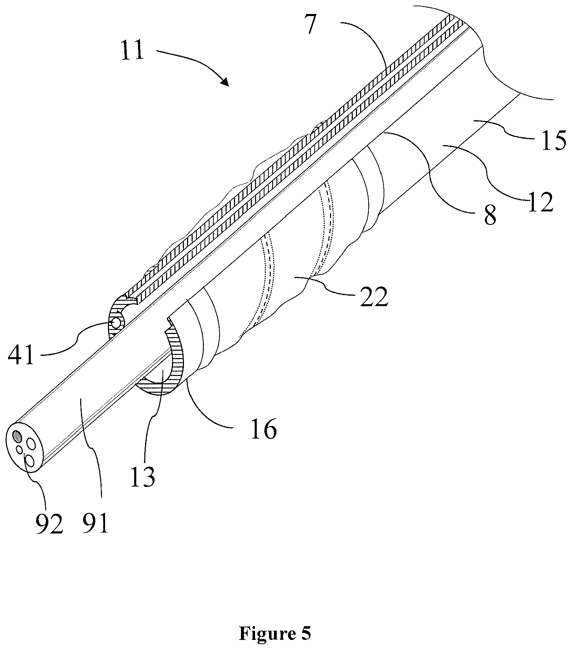

As it is depicted in Figure 2, the overtube 11 of the endoscope accessory 10 comprises of a flexible elongated sheet 1, sized to removably envelop the flexible endoscope or echoendoscope shaft 91, having face 2 and face 3, a proximal end portion 4, a midportion 5, a distal end portion 6, and opposed longitudinal edge portions 7 and 8 that can coact to form a flexible overtube 11 (figure 1). Face 3 forms inner surface 13 (shown in Figure 5) of the overtube 11 which defines a central through passageway for receiving therewithin the endoscope or echoendoscope shaft 91 which can be inserted inside a body cavity such as gastrointestinal tract (figure 1).

As it is depicted in Figure 2, the sheet 1, which forms overtube 11, is further provided with a strip 9 on face 2 of the sheet 1, extending across the entire width of the sheet 1 at the proximal end portion 4 of the sheet 1. In the overtube 11, strip 9 forms handle 19 on the external surface 12 of the flexible overtube 11 when the opposed longitudinal edge portions 7 and 8 coact to form a the flexible overtube 11 (Figure 1).

As it is depicted in Figure 2, the opposed longitudinal edge portions 7 and 8 can be supplied with an adhesive 52 covered by a release sheet 53. After removal of the release sheet 53 from the adhesive 52 at the opposed longitudinal edge portions 7 and 8, the opposed longitudinal edge portions 7 and 8 can be overlapped to form the longitudinal seam 51 along the entire length of the overtube 11 (Figure 1).

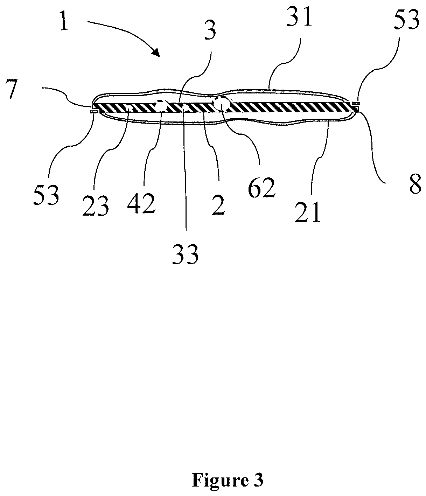

As it is depicted in Figure 3, the sheet 1 is further provided with a catheter passageway 62 and a fluid conduit 42.

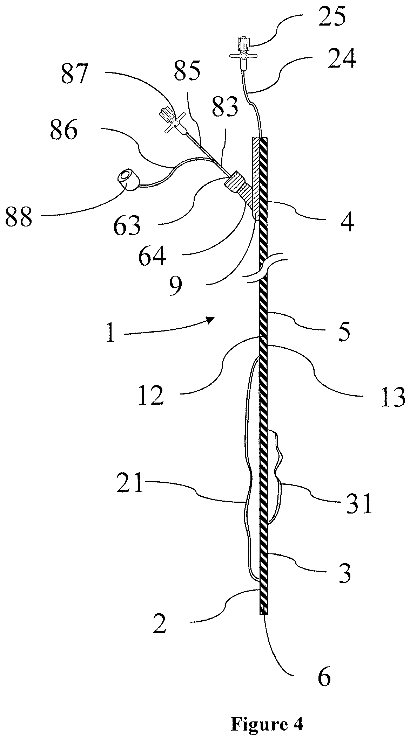

As it is depicted in Figure 4, the catheter passageway 62 extends proximally as a catheter passageway port projection 64 in a projection over the strip 9 at the proximal end portion 4 of the sheet 1. The catheter passageway 62 terminates in a catheter passageway entrance port 63 at the tip of the catheter passageway port projection 64. The catheter passageway 62 defines a through passageway for receiving therewithin a catheter or other endoscopic accessory devices. After the opposed longitudinal edge portions 7 and 8 coact to form the flexible overtube 11, the catheter passageway 62 can be used for passing a catheter or an endoscope accessory device by the overtube 11. A catheter or endoscopic accessory device is inserted into the catheter passageway entrance port 63 on catheter passageway port projection 64 and passed through the catheter passageway 62 and exit from the catheter passageway exit port 61 (shown in Figure 8) inside the body cavity, such as the gastrointestinal tract.

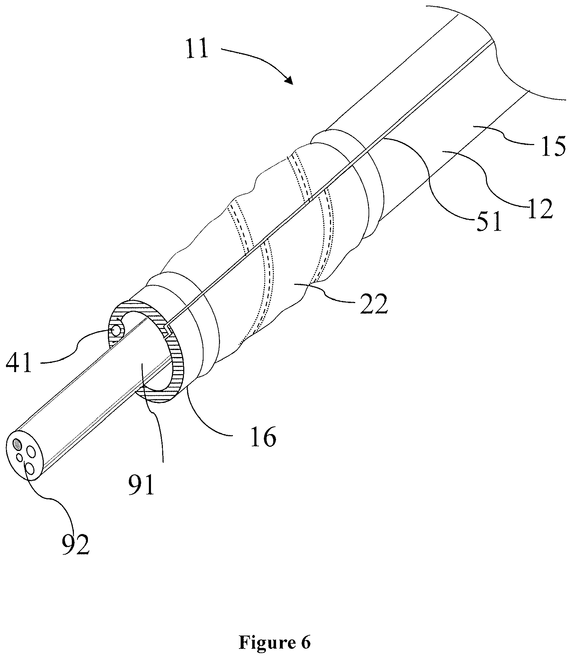

Fluid conduit 42 proximally extends beyond the strip 9 at the proximal end portion 4 of the sheet 1 in the form of a fluid conduit catheter 43 (Figure 2) that terminates in a fluid conduit connection piece 44 that can be used to connect the fluid conduit 42 to an inflation or deflation device. The fluid conduit 42 terminates distally in a fluid conduit port 41 at the distal end portion 16 of the overtube 11 (shown in figure 6). Fluid conduit 42 can be used to inflate or deflate at the distal end portion 16 of the overtube 11.

An inflatable pocket 21 on face 2 of the sheet 1, extends across the entire width of the sheet 1 at the distal end portion 6 of the sheet 1, forming an inflatable positioning ring 22 (Figure 1) on the external surface 12 of the flexible overtube 11.

As it is depicted in Figure 2, 3 and 4, an inflatable band 31 on the face 3 of the sheet 1, extends across the entire width of the sheet 1 and can be situated at either the distal end portion 6 or midportion 5 or proximal end portion 4 of the sheet 1 to form an inflatable sealing band 32 (Figure 9) on the internal surface 13 of the flexible overtube 11. Based on the position of the inflatable band 31 on the sheet 1, which could be either the distal end portion 6 or midportion 5 or proximal end portion 4 of the sheet 1, the inflatable sealing band 32 is situated at the distal end portion 16, midportion 15 or proximal end portion 14 of the overtube 11, respectively.

As it is depicted in Figure 5, the sheet 1 can envelop the endoscope shaft 91 while the opposed longitudinal edge portions 7 and 8 are still apart. When the opposed longitudinal edge portions 7 and 8 coact, the longitudinal seam 51 assembles (figure 6) along entire length of the flexible overtube 11. The overtube provides the external 12 and an internal 13 surface.

As it is depicted in Figure 6, after the longitudinal seam 51 assembles, the overtube 11 forms around the endoscope shaft 91.

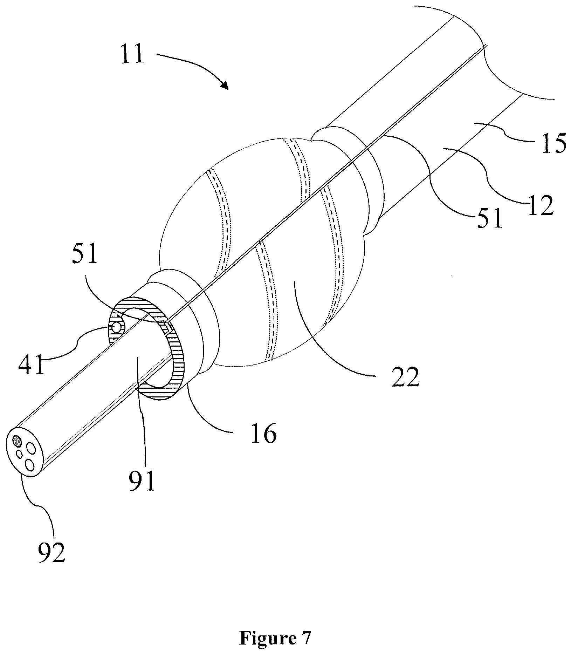

As it is depicted in Figure 7 the inflatable positioning ring 22 at the distal end portion 16 of the overtube 11 can be inflated.

As it is depicted in Figure 8, the inflated positioning ring 22 is asymmetric.

As it is depicted in Figure 9, the inflated positioning ring 22 is eccentric relative to the longitudinal axis of the overtube in a way that the inflatable positioning ring 22 and overtube external surface 12 create internal tangent circles when the inflatable positioning balloon 22 is inflated. The tangent point of these two circles is at the longitudinal seam 51. This allows better sealing created by inflatable positioning balloon 22 within the gastrointestinal lumen and also creates an eccentric position for the endoscope shaft 91 within the gastrointestinal lumen. This eccentric position of the endoscope shaft 91 improves the maneuverability of the endoscope tip 92 within the gastrointestinal lumen when the overtube 11 is rotated.

The inflated sealing band 32 is eccentric relative to the longitudinal axis of the overtube in a way that the inflatable sealing band 32 and overtube internal surface 13 create internal tangent circles when the inflatable sealing band 32 is inflated. The tangent point of these two circles is at the longitudinal seam 51. This allows a better sealing created by inflatable sealing band 32 over the endoscope shaft 91.

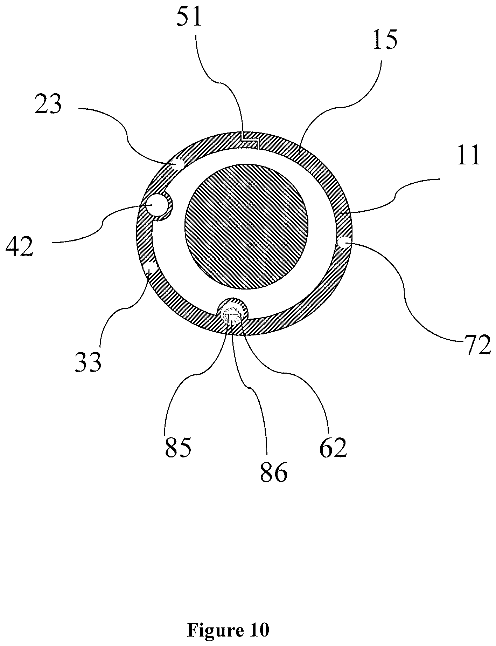

The inflatable positioning ring 22 can be inflated using a positioning ring inflation tube 23 (figure 10). The overtube 11 carries the positioning ring inflation tube 23 that connects distally to the inflatable positioning ring 22 and proximally extends beyond the handle 19 at the proximal end portion 14 of the overtube 11 in the form of an external positioning ring inflation catheter 24 (figure 1) that terminates in a positioning ring inflation stopcock valve 25 (Figure 1) that can be used to inflate or deflate the inflatable positioning ring 22.

As it is depicted in Figure 9, the inflatable sealing band 32 can be inflated using a sealing band inflation tube 33 (figure 10). The overtube 11 carries the sealing band inflation tube 33 that connects distally to the inflatable sealing band 32 and proximally extends beyond the handle 19 at the proximal end portion 14 of the overtube 11 in the form of an external sealing band inflation catheter 34 (figure 1) that terminates in a sealing band inflation stopcock valve 35 (Figure 1) that can be used to inflate or deflate the inflatable sealing band 32.

As it is depicted in Figure 10, the overtube 11 carries several tubes, conduits and a passageway in its midportion 15. One of the conduits is the suction conduit 72. Suction conduit 72 terminates distally at a suction conduit port 71 (shown in Figures 1 and 8) and proximally extends beyond the handle 19 at the proximal end portion 14 of the overtube 11 as an external suction conduit catheter 73 that terminates in a suction conduit connection piece 74 that can be used to connect the suction conduit 72 to a suction device. After the opposed longitudinal edge portions 7 and 8 coact to form a flexible overtube 11, the suction conduit 72 can be used to drain air or water within the body cavity proximal to the positioning ring 22.

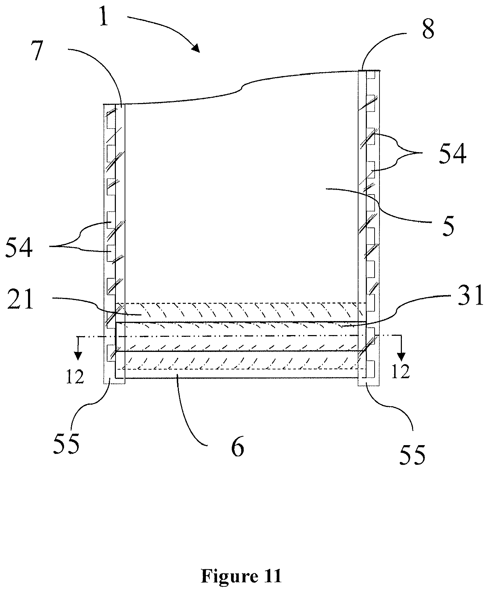

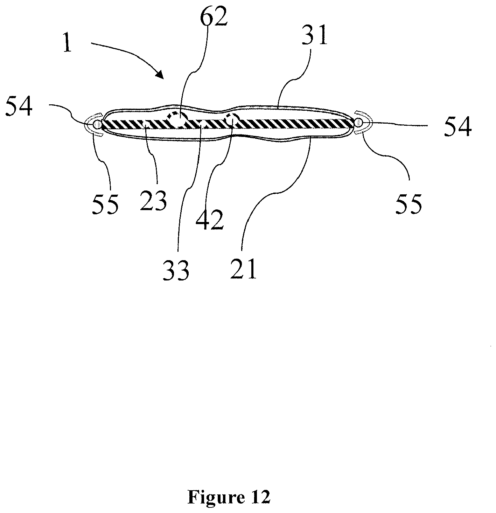

An alternative embodiment in sheet 1 is depicted in Figures 11 and 12. A plurality of spaced magnets 54 are provided in lieu of adhesive 52 and release sheet 53, carried by each longitudinal edge portions 7 and 8 along entire length of the sheet 1 for coacting with the opposed edge portion. The spaced magnets 54 on the longitudinal edge portions 7 are interspersed with the spaced magnets on the longitudinal edge portion 8. The inflatable pocket 21 and the inflatable band 31 are offset from the magnets 54. In order to avoid activation of the magnet before placing the endoscope shaft 91 within the sheet 1, the longitudinal edge portions 7 and 8 is supplied with a longitudinal slit sleeve magnet cover 55. The magnets 54 can only coact with the opposed longitudinal edge portion magnets when the magnet covers 55 are removed from the longitudinal edge portions 7 and 8.

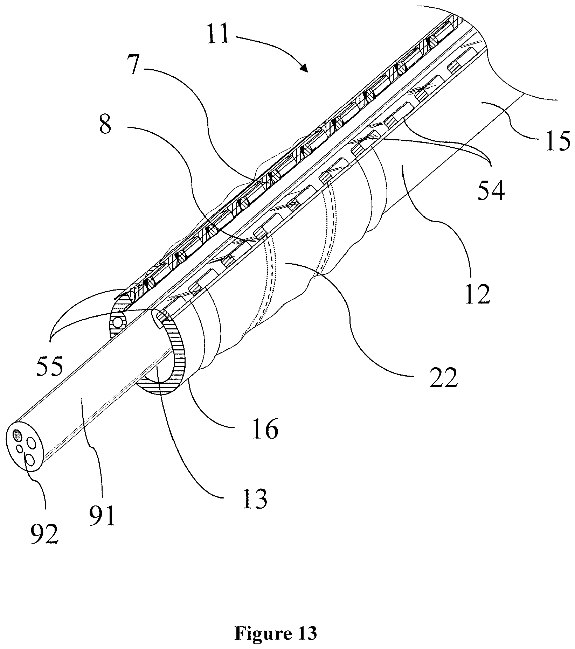

As it is depicted in Figure 13, the sheet 1 can wrap aroundor envelop the endoscope shaft 91 while the opposed longitudinal edge portions 7 and 8 are still apart. The magnets 54 cannot coact with the opposed longitudinal edge portion magnets while the magnet covers 55 are covering the magnets 54 supplied at the longitudinal edge portions 7 and 8.

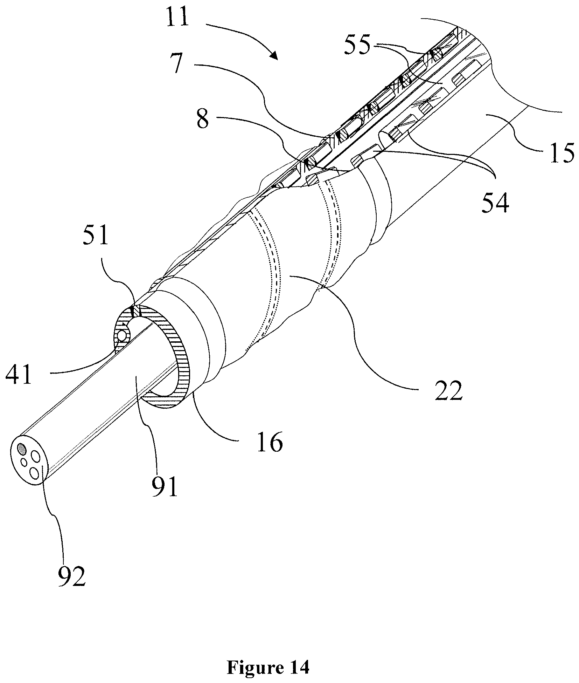

As it is depicted in Figure 14, when the magnet cover 55 is removed by pulling the magnet cover 55 proximally, the distal end portion 16 of the overtube 11 magnets 54 at the opposed longitudinal edge portions 7 and 8 are exposed. The exposed magnets 54 assemble the longitudinal seam 51 starting from the distal end portion 16 of the overtube 11. Repeating this sequence closes the longitudinal seam 51 along entire length of the flexible overtube 11 and the overtube 11 forms around the endoscope shaft 91.

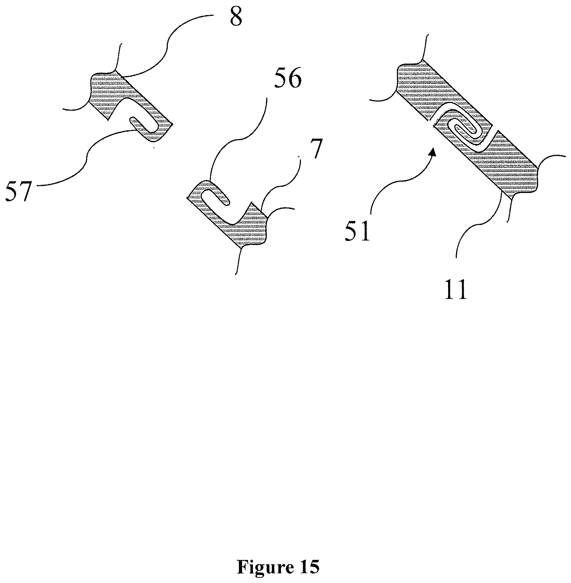

An alternative embodiment in sheet 1 is depicted in Figure 15. Cooperating coupling structures 56 and 57 are provided in lieu of adhesive 52 and release sheet 53, carried by each longitudinal edge portions 7 and 8 along entire length of the sheet 1. The cooperating coupling structures 56 and 57 provide an interlocking closure mechanism in a tongue and groove format and allows a reversible closure or release of the longitudinal seam 51 along the entire length of the overtube 11.

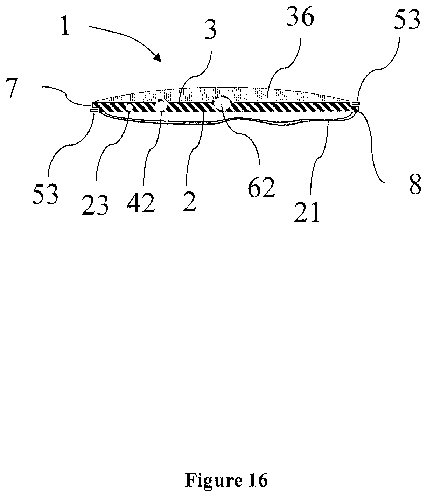

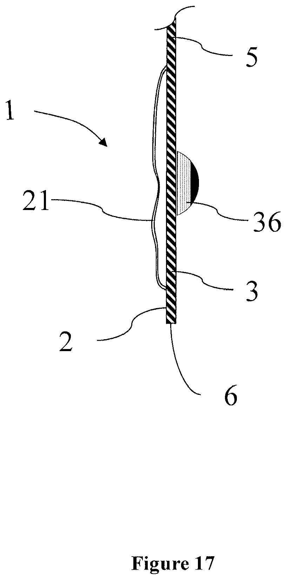

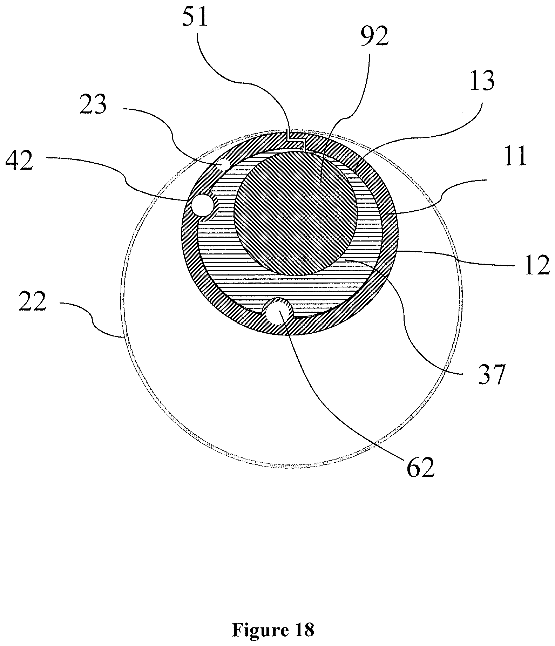

An alternative embodiment in sheet 1 is depicted in Figures 16 and 17. An elongated elastomeric bead 36 on the face 3 of said sheet 1 is provided in lieu of the inflatable pocket 31, extending across entire width of the sheet at the distal end portion 16 of the sheet 1. The elastomeric bead 36 forms an elastomeric sealing band 37 on internal surface 13 of the flexible overtube 11 (Figure 18) when the opposed longitudinal edge portions 7 and 8 coact to form the flexible overtube 11.

As it is depicted in Figure 18, the elastomeric sealing band 37 is eccentric in regard to the overtube in a way that the elastomeric sealing band 37 and overtube internal surface 13 create an internal tangent circles when the opposed longitudinal edge portions 7 and 8 coact to form the flexible overtube 11 and the elastomeric sealing band 37 forms on internal surface 13 of the overtube 11. The tangent point of these two circles is at the longitudinal seam 51. This allows a better sealing created by the elastomeric sealing band 37 over the endoscope shaft 91.

### B- Catheter and Occlusion balloon:

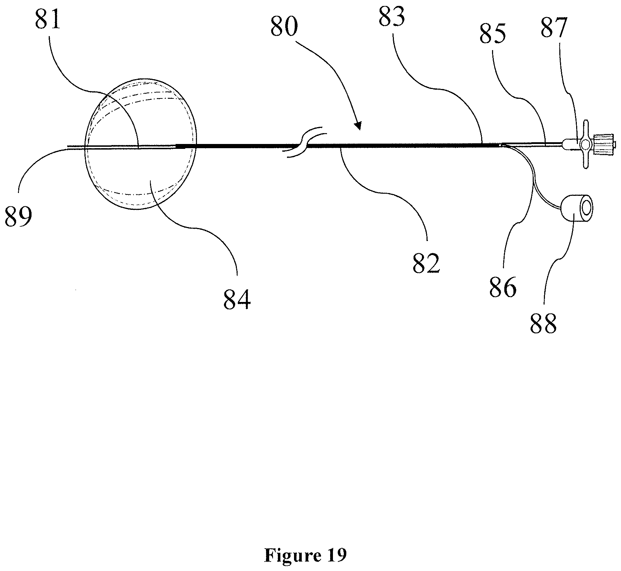

As it is depicted in Figure 19, the occlusion balloon catheter 80 has a distal end portion 81, a midportion 82 and a proximal end portion 83. An occlusion balloon 84 is situated at the distal end portion 81 of the occlusion balloon catheter 80. The occlusion balloon 84 is inflated by an occlusion balloon inflation tube 85 that is carried by the occlusion balloon catheter 80. An occlusion catheter suction tube 86 is also carried by the occlusion balloon catheter 80. At the midportion 82 of the occlusion balloon catheter 80, the occlusion balloon inflation tube 85 and occlusion catheter suction tube 86 are side-by-side. At the proximal end portion 83 of the occlusion balloon catheter 80, the occlusion balloon inflation tube 85 and the occlusion catheter suction tube 86 are separated and terminate at an occlusion balloon inflation stopcock valve 87 and the occlusion catheter suction connection piece 88, respectively. At the distal end portion 81 of the occlusion balloon catheter 80, the occlusion balloon inflation tube 85 terminates at the occlusion balloon 84 and the occlusion catheter suction tube 86 passes through the occlusion balloon 84 to terminate at an occlusion catheter suction tip 89 that can be used to drain air or water within the body cavity distal to the inflatable occlusion balloon 84.

The distal end portion 81 of the occlusion balloon catheter 80 can be independently positioned distal to distal end portion 16 of the overtube 11 within the body cavity. The proximal end portion 83 of the occlusion balloon catheter 81 extends out of the catheter passageway entrance port 63 on a catheter passageway port projection 64 of the overtube 11.

**C- In use,** the endoscope or echoendoscope shaft 91 is inserted to reach to the desired location within a body cavity. At this time, the endoscope accessory sheet 1 is wrapped around the endoscope shaft 91 and opposed longitudinal edge portions 7 and 8 coacting to form a flexible overtube 11 enveloping the shaft of the endoscope 91 over the portion of the shaft 91 that is still outside of the subject's body cavity.

Then, the handle 19 of the overtube 11 is grasped and the distal end portion 16 of the overtube 11 is pushed into the body cavity using the endoscope shaft 91 as a guide. The overtube distal end portion 16 is advanced so that the distal end portion 16 of the overtube 11 reaches to the endoscope tip 92 as it can be seen by endoscope and then, it is pulled back just a few centimeters, so the distal end portion 16 of the overtube 11 is situated just proximal to the endoscope tip 92.

At this point, the inflatable positioning ring 22 at the distal end portion 16 of the overtube 11 is inflated to secure the position of the overtube 11 within the body cavity.

At this point, the endoscope shaft 91 can be removed or replaced with another endoscope, if desired, while the overtube 11 is still within the body cavity.

At this point, the inflatable sealing band 32 of the overtube 11 is inflated to secure the position of the endoscope shaft 91 within the overtube 11.

Then, the distal end portion 81 of the occlusion balloon catheter 80 is inserted into the catheter passageway entrance port 63 on the catheter passageway port projection 64 and passed through the catheter passageway 62 and exit from the catheter passageway exit port 61 at the distal end portion 16 of the overtube 11 inside the body cavity. Then, the inflatable occlusion balloon 84 at the distal end portion 81 of the occlusion balloon catheter 80 can be independently positioned distal to the overtube 11 within the body cavity.

Then, the inflatable occlusion balloon 84 is inflated to secure the position of the distal end portion 81 of the occlusion balloon catheter 80.

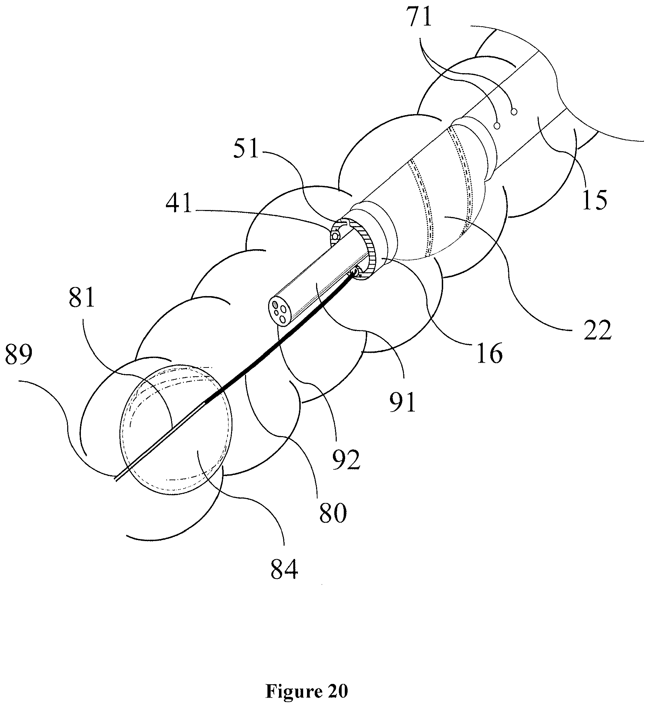

As it is depicted in Figure 20, inflation of the inflatable positioning ring 22, the inflatable sealing band 32 (not shown) and the occlusion balloon 84 defines an examination compartment around the endoscope tip 92. The examination compartment can be filled with air or water depending on the procedure application using the fluid conduit port 41 at the distal end portion 16 of the overtube 11.

The secretions or air at the area proximal to the inflated positioning ring 22 can be aspirated using suction conduit port 71 at the midportion 15 of the overtube 11.

The secretions or air at the area distal to the inflated occlusion balloon 84 can be aspirated using the occlusion catheter suction tip 89, downstream of the occlusion balloon 84.

After completion of the examination, the air or water within the examination compartment is drained via fluid conduit port 41. Then, the inflated positioning ring 22, the inflated sealing band 32 (not shown) and the inflated occlusion balloon 84 are all deflated, and the overtube 11, occlusion balloon catheter 80 as well as the endoscope shaft 91 can be removed independently of each other from the body cavity.

The forgoing description and the drawing are illustrative of the invention and are not to be taken as limiting. Still other variants and rearrangements of structural parts are possible without departing from the scope of this invention and will readily present themselves to those skilled in the art.

## Claims

1. An endoscope accessory (10), which comprises:
a flexible elongated sheet (1), sized to envelop a flexible endoscope or echoendoscope shaft (91), having first and second faces (2, 3), a proximal end portion (14), a midportion (15), a distal end portion (16), and opposed longitudinal edge portions (7, 8) coacting to form a flexible overtube (11) when said longitudinal edge portions (7, 8) overlap, said overtube (11) having a distal endportion (6), a proximal endportion (4), and defining an external surface (12), an internal surface (13), and a central through passageway for receiving therewithin an endoscope or echoendoscope shaft (91);
an inflatable band (31) on the first face (3) of said sheet (1), extending across entire width of the sheet (1) at the distal end portion (6) of the sheet (1), and forming an inflatable sealing band (32) on the internal surface (13) of the flexible overtube (11) when the opposed longitudinal edge portions (7, 8) coact to form the flexible overtube (11);
an inflatable pocket (21) on the second face (2) of the sheet (1), extending across the entire width of the sheet at the distal end portion (6) of the sheet (1), and forming an inflatable positioning ring (22) on the external surface (12) of the flexible overtube (11) when the opposed longitudinal edge portions (7, 8) are joined to form the flexible overtube (11);
an inflation tube (23) in communication with said pocket and provided with an inflation catheter (24); and
a catheter (80) carried by the sheet (1); the catheter (80) having a free, independently positionable distal end portion (81) that terminates in an inflatable occlusion balloon (84); wherein upon inflation of the occlusion balloon (84), the positioning ring (22), and the sealing band (32) create a closed space within a body cavity that is fillable with air and water for improving an examination with the endoscope or the echoendoscope (91);
**characterized in that**
the inflatable positioning ring (22) is asymmetric and wherein the inflatable asymmetric positioning ring (22) and the inflatable sealing band (32) are eccentric relative to the longitudinal axis of the over tube (11).

2. The endoscope accessory (10) in accordance with claim 1 further provided with a strip (9) on the second face (2) of said sheet (1), extending across entire width of the sheet (1) at the proximal end portion (4) of the sheet (1), and forming a handle (19) on the external surface (12) of the flexible overtube (11) when the opposed longitudinal edge portions (7, 8) coact to form the flexible overtube (11).

3. The endoscope accessory (10) in accordance with claim 1 wherein an adhesive is provided on the edge portions of the sheet (1) along entire length thereof and positional to form a liquid tight seam when said edge portions overlap, and wherein the inflatable pocket (21) and the inflatable band (31) are offset from the adhesive.

4. The endoscope accessory (10) in accordance with claim 1 wherein the longitudinal edge portions (7, 8) have a thickness which is less than the thickness of the sheet (1).

5. The endoscope accessory (10) in accordance with claim 1 wherein a plurality of spaced magnets is carried by each longitudinal edge portion (7, 8) of the sheet (1) along the entire length for coacting with the opposed edge portion (7, 8) to form a seam (51), and the inflatable pocket (21) and the inflatable band (32) are offset from the magnets (54).

6. The endoscope accessory (10) in accordance with claim 5 wherein said spaced magnets (54) on one of the longitudinal edge portions (7, 8) are interspersed with the spaced magnets (54) on the other said longitudinal edge portion (7, 8) in said seam (51).

7. The endoscope accessory (10) in accordance with claim 5 wherein said magnets (54) on at least one longitudinal edge portion (7, 8) are provided with a cover (55).

8. The endoscope accessory (10) in accordance with claim 1 wherein the sealing band (32) is asymmetrical.

9. The endoscope accessory (10) in accordance with claim 1 wherein the sheet (1) defines a suction port (71) and a suction conduit (72) that is provided in fluid communication with the suction port (71), said suction port (71) being situated on the second face (2) of the sheet (1) between the inflatable pocket (21) and the proximal end portion (4) of the sheet (1).

10. The endoscope accessory (10) in accordance with claim 1 wherein the catheter (80) is a dual lumen catheter defining an inflation passageway for the occlusion balloon (84) and a suction passageway that terminates in a suction tip downstream from the occlusion balloon (84).

## Patentansprüche

1. Zubehör (10) für ein Endoskop, das Folgendes umfasst:
ein flexibles längliches Flächenstück (1), dass eine solche Größe aufweist, dass es einen flexiblen Endoskop- oder Echoendoskopschaft (91) umhüllt, mit ersten und zweiten Stirnflächen (2, 3), einem proximalen Endabschnitt (14), einem mittleren Abschnitt (15), einem distalen Endabschnitt (16) und gegenüberliegenden länglichen Randabschnitten (7, 8), die zur Bildung eines flexiblen Überrohrs (11) zusammenwirken, wenn sich die länglichen Randabschnitte (7, 8) überlappen, wobei das Überrohr (11) einen distalen Endabschnitt (6) und einen proximalen Endabschnitt (4) aufweist, und eine Außenfläche (12), eine Innenfläche (13) und einen mittigen Durchgang zur Aufnahme eines Endoskop- oder Echoendoskopschafts (91) darin definiert;
ein aufblasbares Band (31) auf der ersten Stirnfläche (3) des Flächenstücks (1), das sich über die gesamte Breite des Flächenstücks (1) am distalen Endabschnitt (6) des Flächenstücks (1) erstreckt, und ein aufblasbares Dichtungsband (32) auf der Innenfläche (13) des flexiblen Überrohrs (11) bildet, wenn die gegenüberliegenden länglichen Randabschnitte (7, 8) zur Bildung des flexiblen Überrohrs (11) zusammenwirken;
eine aufblasbare Tasche (21) auf der zweiten Stirnfläche (2) des Flächenstücks (1), die sich über die gesamte Breite des Flächenstücks am distalen Endabschnitt (6) des Flächenstücks (1) erstreckt, und einen aufblasbaren Positionierungsring (22) auf der Außenfläche (12) des flexiblen Überrohrs (11) bildet, wenn die gegenüberliegenden länglichen Randabschnitte (7, 8) zur Bildung des flexiblen Überrohrs (11) miteinander verbunden werden;
einen Aufblasschlauch (23), das mit der Tasche in Verbindung steht und einen Aufblaskatheter (24) aufweist; und
einen Katheter (80), der von dem Flächenstück (1) getragen wird; wobei der Katheter (80) einen freien unabhängig positionierbaren distalen Endabschnitt (81) aufweist, der in einem aufblasbaren Okklusionsballon (84) endet; wobei beim Aufblasen des Okklusionsballons (84) der Positionierungsring (22) und das Dichtungsband (32) einen geschlossenen Raum innerhalb einer Körperhöhle erzeugen, der mit Luft und Wasser gefüllt werden kann, um eine Untersuchung mit dem Endoskop oder Echoendoskop (91) zu verbessern;
**dadurch gekennzeichnet, dass**
der aufblasbare Positionierungsring (22) asymmetrisch ist und wobei der aufblasbare asymmetrische Positionierungsring (22) und das aufblasbare Dichtungsband (32) bezüglich der Längsachse des Überrohrs (11) exzentrisch sind.

2. Zubehör (10) für ein Endoskop nach Anspruch 1, ferner mit einem Streifen (9) auf der zweiten Stirnfläche (2) des Flächenstücks (1) ausgestattet, der sich über die gesamte Breite des Flächenstücks (1) am proximalen Endabschnitt (4) des Flächenstücks (1) erstreckt, und einen Griff (19) auf der Außenfläche (12) des flexiblen Überrohrs (11) bildet, wenn die gegenüberliegenden länglichen Randabschnitte (7, 8) zur Bildung des flexiblen Überrohrs (11) zusammenwirken.

3. Zubehör (10) für ein Endoskop nach Anspruch 1, wobei ein Klebstoff auf den Randabschnitten des Flächenstücks (1) entlang seiner gesamten Länge und lagerichtig vorgesehen ist, um eine flüssigkeitsdichte Nahtverbindung zu bilden, wenn sich die Randabschnitte überlappen, und wobei die aufblasbare Tasche (21) und das aufblasbare Band (31) vom Klebstoff versetzt sind.

4. Zubehör (10) für ein Endoskop nach Anspruch 1, wobei die länglichen Randabschnitte (7, 8) eine Dicke aufweisen, die kleiner als die Dicke des Flächenstücks (1) ist.

5. Zubehör (10) für ein Endoskop nach Anspruch 1, wobei eine Vielzahl von beabstandeten Magneten von jedem länglichen Randabschnitt (7, 8) des Flächenstücks (1) entlang der gesamten Länge zum Zusammenwirken mit dem gegenüberliegenden Randabschnitt (7, 8) getragen wird, um eine Nahtverbindung (51) zu bilden, und die aufblasbare Tasche (21) und das aufblasbare Band (32) von den Magneten (54) versetzt sind.

6. Zubehör (10) für ein Endoskop nach Anspruch 5, wobei die beabstandeten Magneten (54) auf einem der länglichen Randabschnitte (7, 8) mit den beabstandeten Magneten (54) auf dem anderen besagten länglichen Randabschnitt (7, 8) in der Nahtverbindung (51) durchsetzt sind.

7. Zubehör (10) für ein Endoskop nach Anspruch 5, wobei die Magneten (54) auf mindestens einem länglichen Randabschnitt (7, 8) eine Abdeckung (55) aufweisen.

8. Zubehör (10) für ein Endoskop nach Anspruch 1, wobei das Dichtungsband (32) asymmetrisch ist.

9. Zubehör (10) für ein Endoskop nach Anspruch 1, wobei das Flächenstück (1) eine Saugöffnung (71) und eine Saugleitung (72), die in Fluidverbindung mit der Saugöffnung (71) bereitgestellt ist, definiert, wobei die Saugöffnung (71) auf der zweiten Stirnfläche (2) des Flächenstücks (1) zwischen der aufblasbaren Tasche (21) und dem proximalen Endabschnitt (4) des Flächenstücks (1) angeordnet ist.

10. Zubehör (10) für ein Endoskop nach Anspruch 1, wobei der Katheter (80) ein Doppellumen-Katheter ist, der einen Aufblasdurchgang für den Okklusionsballon (84) und einen Saugdurchgang definiert, der in einer Saugspitze stromabwärts vom Okklusionsballon (84) endet.

## Revendications

1. Accessoire (10) d'endoscope, qui comprend :
une feuille (1) allongée flexible, dimensionnée pour envelopper la gaine souple (91) d'un endoscope ou d'un échoendoscope, comportant une première et une seconde face (2, 3), une partie terminale proximale (14), une partie centrale (15), une partie terminale distale (16), et des parties latérales longitudinales (7, 8) opposées qui coopèrent pour constituer un surtube (11) flexible quand lesdites parties latérales longitudinales (7, 8) se chevauchent, ledit surtube (11) comportant une partie terminale distale (6), une partie terminale proximale (4) et délimitant une surface externe (12), une surface interne (13), et un passage central traversant pour y recevoir la gaine (91) d'un endoscope ou d'un échoendoscope ;
une bande gonflable (31) sur la première face (3) de ladite feuille (1), qui s'étend en travers de toute la largeur de la feuille (1) à la partie terminale distale (6) de la feuille (1) et constitue une bande d'étanchéité (32) gonflable sur la surface interne (13) du surtube (11) flexible quand les parties latérales longitudinales (7, 8) opposées coopèrent pour constituer le surtube (11) flexible ;
une poche gonflable (21) sur la seconde face (2) de la feuille (1), qui s'étend en travers de toute la largeur de la feuille à la partie terminale distale (6) de la feuille (1) et constitue un anneau de mise en place (22) gonflable sur la surface externe (12) du surtube (11) flexible quand les parties latérales longitudinales (7, 8) opposées sont assemblés pour constituer le surtube (11) flexible ;
un tube de gonflage (23) en communication avec ladite poche et pourvu d'un cathéter de gonflage (24) ; et
un cathéter (80) porté par la feuille (1), le cathéter (80) comportant une partie terminale distale (81) libre, pouvant être mise en place indépendamment et qui se termine par un ballonnet d'occlusion (84) gonflable,
dans lequel, lorsqu'on gonfle le ballonnet d'occlusion (84), l'anneau de mise en place (22) et la bande d'étanchéité (32) créent un espace clos à l'intérieur d'une cavité corporelle qu'on peut remplir d'air et d'eau pour améliorer un examen à l'aide de l'endoscope ou de l'échoendoscope (91),
**caractérisé en ce que** l'anneau de mise en place (22) est asymétrique et
dans lequel l'anneau de mise en place (22) gonflable et la bande d'étanchéité (32) gonflable sont excentrés par rapport à l'axe longitudinal du surtube (11).

2. Accessoire (10) d'endoscope selon la revendication 1, comprenant en outre un ruban (9) sur la seconde face (2) de ladite feuille (1), qui s'étend en travers de toute la largeur de la feuille (1) à la partie terminale proximale (4) de la feuille (1) et constitue une poignée (19) sur la surface externe (12) du surtube (11) flexible quand les parties latérales longitudinales (7, 8) opposées coopèrent pour constituer le surtube (11) flexible.

3. Accessoire (10) d'endoscope selon la revendication 1, dans lequel un adhésif est disposé sur les parties latérales de la feuille (1) sur toute la longueur de celle-ci et mis en place pour constituer un joint étanche aux liquides quand lesdites parties latérales se chevauchent, et ///dans lequel la poche gonflable (21) et la bande gonflable (31) sont déportées de l'adhésif.

4. Accessoire (10) d'endoscope selon la revendication 1, dans lequel les parties latérales longitudinales (7, 8) ont une épaisseur qui est inférieure à l'épaisseur de la feuille (1).

5. Accessoire (10) d'endoscope selon la revendication 1, dans lequel une pluralité d'aimants espacés sont portés par chaque partie latérale longitudinale (7, 8) de la feuille (1) sur toute la longueur pour coopérer avec la partie latérale (7, 8) afin de constituer un joint (51), et la poche gonflable (21) et la bande gonflable (32) sont déportées des aimants (54).

6. Accessoire (10) d'endoscope selon la revendication 5, dans lequel lesdits aimants (54) espacés sur une des parties latérales longitudinales (7, 8) sont intercalés avec les aimants (54) espacés sur l'autre des parties latérales longitudinales (7, 8) dans ledit joint (51).

7. Accessoire (10) d'endoscope selon la revendication 5, dans lequel lesdits aimants (54) sur au moins une partie latérale longitudinale (7, 8) sont pourvus d'un couvercle (55).

8. Accessoire (10) d'endoscope selon la revendication 1, dans lequel la bande d'étanchéité (32) est asymétrique.

9. Accessoire (10) d'endoscope selon la revendication 1, dans lequel la feuille (1) délimite un orifice d'aspiration (71) et un conduit d'aspiration (72) qui est disposé en communication fluidique avec l'orifice d'aspiration (71), ledit orifice d'aspiration (71) étant situé sur la seconde face (2) de la feuille (1) entre la poche gonflable (21) et la partie terminale proximale (4) de la feuille (1).

10. Accessoire (10) d'endoscope selon la revendication 1, dans lequel le cathéter (80) est un cathéter à double lumière délimitant un passage de gonflage pour le ballonnet d'occlusion (84) et un passage d'aspiration qui se termine par un embout d'aspiration en aval du ballonnet d'occlusion (84).
